# EUROPEAN PATENT SPECIFICATION

(11) **EP 4 272 771 B1**
(45) Date of publication and mention of the grant of the patent: **28.10.2026**
(21) Application number: 21914434.2
(22) Date of filing: 28.12.2021
(51) Int. Cl.: A61L 27/36, A61L 27/50

(54) **FOLD RESISTANT DEHYDRATED CROSS-LINKED BIOLOGICAL MATERIAL, PREPARATION METHOD THEREFOR, AND APPLICATION THEREOF**
FALZRESISTENTES DEHYDRIERTES VERNETZTES BIOLOGISCHES MATERIAL, HERSTELLUNGSVERFAHREN DAFÜR UND ANWENDUNG DAVON
MATÉRIAU BIOLOGIQUE RÉTICULÉ DÉSHYDRATÉ RÉSISTANT AU PLIAGE, PROCÉDÉ DE PRÉPARATION ASSOCIÉ ET APPLICATION ASSOCIÉE

(30) Priority: 31.12.2020 CN 202011621441
(43) Date of publication of application: 08.11.2023
(73) Proprietor: Venus MedTech (HangZhou), Inc., Hangzhou, Zhejiang 310052 (CN)
(72) Inventor: WEI, Xing, Hangzhou, Zhejiang 310052 (CN); LIM, Hou-Sen, Hangzhou, Zhejiang 310052 (CN); KUANG, Dajun, Hangzhou, Zhejiang 310052 (CN)
(74) Representative: KIPA AB
(86) International application number: PCT/CN2021/142206
(87) International publication number: WO 2022/143700

(56) References cited:
- WO-A2-2011/160085
- CN-A- 103 313 735
- CN-A- 103 705 980
- CN-A- 109 260 517
- CN-A- 109 821 070
- CN-A- 109 833 118
- CN-A- 111 701 079
- JP-A- 2002 143 290
- US-A1- 2002 081 564

## Description

### TECHNICAL FIELD

The present application relates to the technical field of prosthetic biological materials, in particular to a crease resistant dehydrated cross-linked biological material preparation method therefor and application thereof.

### DESCRIPTION OF THE PRIOR ART

In recent years, interventional biological material (such as interventional valve) replacement surgery has a wide clinical application due to its convenient operation and reducing the secondary damage to patients greatly. At present, interventional valves are usually preserved in a glutaraldehyde solution. In practice, they need to be washed repeatedly for loading, increasing the surgery time and additional risks. Further, the residual glutaraldehyde on the biological valve may increase the calcification and toxicity of the biological valve. These problems can be overcome by dehydrating and preloading the biological valve. However, using dehydrating and preloading, the biological valve, which is then hydrated after being crimped for a long time, needs to have good elasticity and toughness so as to avoid creases after long-term crimping. Currently, biological valves still cannot overcome the problem of creases after long-term crimping. The creases not only affect the hydrodynamic properties of the valve, but also are more likely to cause calcification and fatigue tear. PCT Publication WO 2011160085 A2 discloses methods for modifying antigenic carbohydrate epitopes within a xenographic bioprosthetic tissue by oxidation of vicinal diols to form aldehydes or acids and subsequence reductive animation of aldehydes to form stable secondary amines, or amidation or esterification of acids to form stable amides or esters. US Patent Publication No. 2002081564 A1 discloses methods of stabilizing glycosaminoglycans in a biological tissue (e.g. a bioprosthetic implant) in conjunction with cross-linking of protein in the tissue.

### SUMMARY OF THE DISCLOSURE

The invention is defined by the appended claims. Further, the present disclosure provides a crease resistant dehydrated cross-linked biological material and the preparation method therefor and the application thereof, which solve the problem that the creases on a dry valve being crimped for a long time in a delivery device cannot be recovered after rehydration.

A method for preparing a crease resistant dehydrated biological material through composite crosslinking, includes performing (a) oxidation, (b) first crosslinking, (c) second crosslinking and (d) dehydration on the biological material, wherein (b) is performed after (a), and (a), (b) and (c) are all performed prior to (d).

The oxidation uses an oxidant capable of converting hydroxyl groups of mucopolysaccharides of the biological material into aldehyde groups, the first crosslinking uses a first crosslinking agent capable of causing cross-linking between the aldehyde groups of the mucopolysaccharides, and the second crosslinking uses a second crosslinking agent capable of causing cross-linking between collagen fibers of the biological material.

Optionally, the collagen of the biological material has a content ranging from 60% to 90%.

Optionally, the biological material is originated from pig, cattle, horse or sheep, and selected from pericardium, heart valve, blood vessel, ligament, muscle, intestine or skin.

Further optionally, the biological material includes porcine pericardium or bovine pericardium.

Optionally, the oxidation includes exposing the biological material into a solution containing the oxidant.

Optionally, the hydroxyl group is an ortho-hydroxyl group from the mucopolysaccharide.

Optionally, the oxidant is sodium periodate.

Optionally, the oxidant in the solution containing the oxidant has a mass percentage concentration ranging from 0.1% to 1%. The solvent for preparing the oxidant solution is a conventional solvent for treating biological material, which will not destroy the structure of the biological material, such as water, PBS or physiological saline etc.

Optionally, the method includes exposing the biological material into the solution containing the oxidant for 1 to 12 hours.

Optionally, the method includes exposing the biological material into the solution containing the oxidant by static contact or dynamic contact.

Static contact refers to that the biological material and the solution containing the oxidant keep static relative to each other during the oxidation, such as by soaking. Dynamic contact refers to that the biological material and the solution containing the oxidant are movable relative to each other during the oxidation, such as by spraying the solution containing the oxidant repeatedly to the biological material, or stirring or shaking the reaction system when the biological material is soaked in the solution containing the oxidant. The static contact or dynamic contact mentioned below can be explained in the same way unless otherwise specified.

The biological material is exposed into the solution containing the oxidant, where the oxidant is used to oxidize the ortho-hydroxyl groups of the mucopolysaccharides in the biological material into aldehyde groups, and the mucopolysaccharides are connected to the collagen fibers in the biological material through the oxidized aldehyde groups. The oxidized biological material is exposed into the first crosslinking agent for crosslinking, where the mucopolysaccharides in the biological material are cross-linked by the first crosslinking agent. The biological material is exposed into the second crosslinking agent for crosslinking, where the collagen fibers in the biological material are cross-linked by the second crosslinking agent. The cross-linked biological material is dehydrated or dried.

The biological material is exposed to a solution containing an oxidant, where the oxidant oxidizes the ortho-hydroxyl groups of the mucopolysaccharide molecules in the biological material into aldehyde groups, and the oxidized aldehyde groups are cross-linked with the amino groups of the collagen fibers in the biological material. In the further crosslinkings, mucopolysaccharide molecules, and collagen fibers are respectively cross-linked by corresponding crosslinking agents to form a network. The glycosyl groups in the biological material are opened in situ and form part of the cross-linked network. The elasticity and water absorption of the obtained cross-linked biological material are significantly improved, and the biological material, although being crimped for a long time in the delivery device, has no obvious creases after rehydration. Further, the mucopolysaccharide molecules are part of the biological material, which open in situ and form part of the cross-linked network, solving the problems of both crease recovery and immunogenicity.

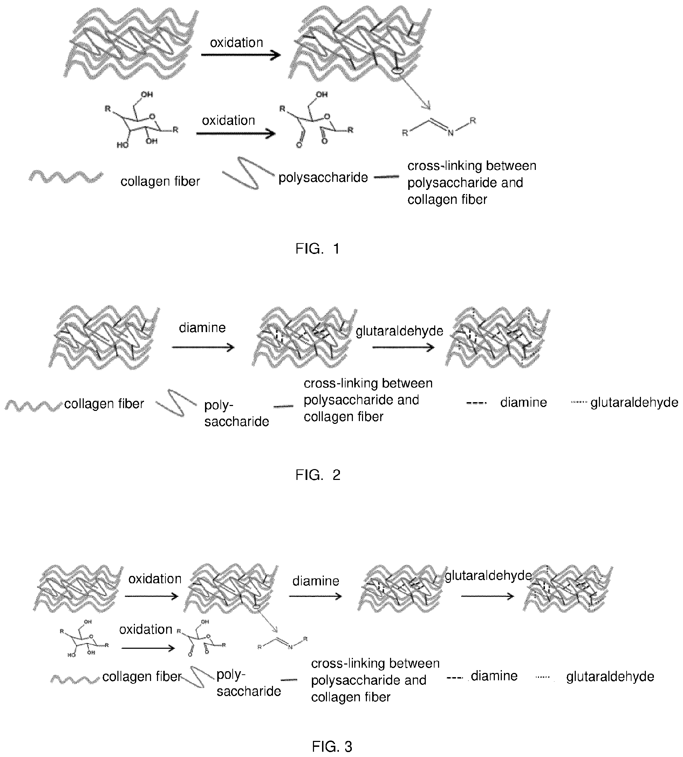

The main reaction occurred in the oxidation is shown in FIG. 1. In the preparation methods performed in the order of (a)(b)(c), (a)(c)(b) or (c)(a)(b) (not part of the invention), the steps of the respective oxidations are the same.

An optional treating method is provided: performing (a) oxidation, (b) first crosslinking and (c) second crosslinking on the biological material in sequence by exposing the biological material after oxidation to a first crosslinking agent solution and a second crosslinking agent solution.

Optionally, the first crosslinking agent has at least two active groups capable of reacting with the aldehyde groups, and the second crosslinking agent is different from the first crosslinking agent and capable of reacting with the active groups of the first crosslinking agent.

In this treating method, the crosslinking between mucopolysaccharides is performed first, and then the crosslinking between collagen fibers is performed. In the oxidation, the ortho-hydroxyl groups of mucopolysaccharides in the biological material are oxidized to aldehyde groups, and some aldehyde groups react with the amino groups of collagen fibers in the biological material, thereby causing crosslinking between mucopolysaccharide and collagen fiber. The active groups of the first crosslinking agent react with the remaining aldehyde groups of mucopolysaccharides, thereby causing crosslinking between mucopolysaccharides. The first crosslinking agent for cross-linking the aldehyde groups of mucopolysaccharide introduces some free active groups. The second crosslinking agent mainly reacts with the amino groups or carboxyl groups of the collagen fibers, thereby causing crosslinking between the collagen fibers. Further, the free active groups introduced by the previous crosslinking are also cross-linked by the second crosslinking agent so that the step of blocking the residual active groups of the first crosslinking agent can be omitted.

Optionally, the first crosslinking agent is a small molecular substance having at least two amino groups.

The small molecular substance having at least two amino groups includes amino acid, diamine, etc.. The amino acid can be lysine. The diamine can be an amino compound containing two amino groups, such as ethylenediamine, propylenediamine, hexamethylenediamine, p-phenylenediamine, etc.

Optionally, the first crosslinking agent is lysine, ethylenediamine or hexamethylenediamine.

Optionally, the first crosslinking agent in the first crosslinking agent solution has a mass percentage concentration ranging from 0.05% to 5%. The solvent for preparing the first crosslinking agent solution is a conventional solvent for treating biological material, which will not destroy the structure of the biological material, such as water, PBS or physiological saline etc.

Optionally, the method includes exposing the biological material into the first crosslinking agent solution for 0.5 to 12 hours.

Optionally, the second crosslinking agent is glutaraldehyde, and the second crosslinking agent in the second crosslinking agent solution has a mass percentage concentration ranging from 0.05% to 25%. The solvent for preparing the second crosslinking agent solution is a conventional solvent for treating biological material, which will not destroy the structure of the biological material, such as water, PBS or physiological saline etc.. The crosslinking time ranges from 6 h to 3 weeks, and the crosslinking temperature ranges from 0 to 37 °C.

Optionally, the method includes exposing the biological material into the first crosslinking agent solution and the second crosslinking agent solution by static contact or dynamic contact.

In this crosslinking method, for example, taking diamine as the first crosslinking agent and glutaraldehyde as the second crosslinking agent, the reaction mechanism is shown in FIG. 2, where the diamine cross-links the mucopolysaccharide molecules, and the glutaraldehyde cross-links the collagen fibers.

Taking diamine as the first crosslinking agent and glutaraldehyde as the second crosslinking agent, for example, the whole reaction mechanism of oxidation and crosslinkings in two steps is shown in FIG. 3. In the oxidation, the oxidant oxidizes the ortho-hydroxyl groups of the mucopolysaccharide molecules in the biological material into aldehyde groups, and the oxidized aldehyde groups are cross-linked with the amino groups of the collagen fibers in the biological material. In the crosslinking step with the first crosslinking agent, the first crosslinking agent, such as diamine, connects the mucopolysaccharide molecules in the biological material through the reaction of amino groups and aldehyde groups. In the crosslinking step with the second crosslinking agent, the second crosslinking agent, such as glutaraldehyde, connects the collagen fibers in the biological material through the reaction of the aldehyde groups and the amino groups or the carboxyl groups, thereby forming a final cross-linked network. In the cross-linked network, mucopolysaccharide molecules are linked with collagen fibers, mucopolysaccharide molecules are linked to each other, and collagen fibers are linked to each other. The mucopolysaccharide molecules of the biological material forming part of the cross-linked network have a good hydrophilic property, which improves the hydrophilicity of the biological material so that the biological material can quickly become flattened after absorbing water.

After crosslinking with the first crosslinking agent (diamine), the residual amino groups from the first crosslinking agent can be cross-linked by the second crosslinking agent during the crosslinking with the second crosslinking agent, so that the step of treating the residual amino groups from the first crosslinking agent can be omitted.

Optionally, the method further includes end-capping of exposing the cross-linked biological material into a solution containing a capping substance to block residual active groups from the second crosslinking agent.

The active groups from the crosslinking agent can be understood as coming from the corresponding crosslinking agent. In this method, the active groups from the crosslinking agent are the active groups coming from the second crosslinking agent.

Optionally, the capping substance is a polyamino substance.

Optionally, the polyamino substance is polyethyleneimine, polylysine, ε-polylysine, lysine or hexamethylenediamine.

Optionally, the solvent for preparing the solution containing the capping substance is a conventional solvent for treating biological material, which will not destroy the structure of the biological material, such as water, PBS or physiological saline etc.. The capping substance in the solution containing the capping substance has a mass percentage concentration ranging from 0.5% to 10%.

Optionally, the method includes exposing the biological material into the solution containing the capping substance for 0.5 h to 12 h.

Optionally, the method includes exposing the biological material into the solution containing the capping substance by static contact or dynamic contact.

Optionally, the method further includes post-crosslinking of exposing the capped biological material into a third crosslinking agent solution to crosslink residual active groups from the capping substance.

In the end-capping step, the capping substance is a polyamino substance. The polyamino substance includes at least two amino groups, some of which react with the residual aldehyde groups, with the remaining amino groups as residual amino groups. The polyamino substance has a high content of amino groups and thus a high content of residual amino groups, so that there are many sites for crosslinking with the third crosslinking agent in the post-crosslinking step, and the resulted cross-linked network can further enhance the elasticity of the biological material.

Optionally, the third crosslinking agent in the third crosslinking agent solution has at least one active group capable of reacting with amino group(s) and one hydrophilic group.

Optionally, the group capable of reacting with amino group(s) is an ester bond or an epoxy group, and the hydrophilic group is an alcoholic hydroxyl group or an ether bond.

Optionally, the third crosslinking agent is polyethylene glycol diglycidyl ether.

Optionally, the solvent for preparing the third crosslinking agent solution is a conventional solvent for treating biological material, which will not destroy the structure of the biological material, such as water, PBS or physiological saline etc.. The third crosslinking agent in the third crosslinking agent solution has a mass percentage concentration ranging from 0.01% to 2%.

Optionally, the method further includes exposing the biological material into the third crosslinking agent solution for 2 h to 48 h.

Optionally, the method further includes exposing the biological material into the third crosslinking agent solution by static contact or dynamic contact.

Also disclosed (not part of the invention) is performing (c) second crosslinking, (a) oxidation and (b) first crosslinking on the biological material in sequence by exposing the biological material into a second crosslinking agent solution, a solution containing the oxidant and a first crosslinking agent solution.

Another optional treating method is provided: performing (a) oxidation, (c) second crosslinking and (b) first crosslinking on the biological material in sequence by exposing the biological material into a solution containing the oxidant, a second crosslinking agent solution and a first crosslinking agent solution.

Optionally, the second crosslinking agent has at least two active groups capable of reacting with amino group(s) or carboxyl group(s), and the first crosslinking agent is different from the second crosslinking agent and capable of reacting with the active groups of the second crosslinking agent.

In these treating methods, the crosslinking of collagen fibers is performed first, and then the crosslinking of mucopolysaccharides is performed. In the oxidation, the ortho-hydroxyl groups of mucopolysaccharides in the biological material are oxidized to aldehyde groups, and some aldehyde groups are cross-linked with the amino groups of collagen fibers in the biological material. The second crosslinking agent reacts with the amino groups or carboxyl groups of the collagen fibers, thereby causing crosslinking between the collagen fibers. The second crosslinking agent also introduces some free active groups while crosslinking with the collagen fibers. The first crosslinking agent mainly reacts with the aldehyde groups of mucopolysaccharides, thereby causing crosslinking between mucopolysaccharides. The free active groups introduced by the crosslinking step with the second crosslinking agent are also cross-linked by the first crosslinking agent so that the step of blocking the residual active groups of the first crosslinking agent can be omitted.

Optionally, the first crosslinking agent is a small molecular substance having at least two amino groups.

The small molecular substance having at least two amino groups includes amino acid, polyamine, etc.. The amino acid can be selected from lysine, polylysine or ε-polylysine, etc.. The polyamine can be diamine being an amino compound with two amino groups, for example, ethylenediamine, propylenediamine, hexamethylenediamine, p-phenylenediamine, etc. Polyethyleneimine can also be used as the polyamine.

Optionally, the first crosslinking agent is lysine, ethylenediamine, hexamethylenediamine, polyethyleneimine, polylysine or ε-polylysine.

Optionally, the first crosslinking agent in the first crosslinking agent solution has a mass percentage concentration ranging from 0.5% to 10%. The solvent for preparing the first crosslinking agent solution is a conventional solvent for treating biological material, which will not destroy the structure of the biological material, such as water, PBS or physiological saline etc..

Optionally, the method includes exposing the biological material into the first crosslinking agent solution for 0.5 to 12 hours.

Optionally, the second crosslinking agent is glutaraldehyde, and the second crosslinking agent in the second crosslinking agent solution has a mass percentage concentration ranging from 0.05% to 25%. The solvent for preparing the second crosslinking agent solution is a conventional solvent for treating biological material, which will not destroy the structure of the biological material, such as water, PBS or physiological saline etc.. The crosslinking time ranges from 6 h to 3 weeks, and the crosslinking temperature ranges from 0 to 37 °C.

Optionally, the method includes exposing the biological material into the first crosslinking agent solution and the second crosslinking agent solution by static contact or dynamic contact.

Optionally, the method further includes post-crosslinking of exposing the biological material treated with the first crosslinking agent into a third crosslinking agent solution to crosslink residual active groups from the first crosslinking agent.

In this treating method, when the first crosslinking agent uses a bisamino small molecular substance, the residual active groups after the crosslinking with the first crosslinking agent are amino groups, and the post-crosslinking step with polyethylene glycol can be directly performed.

Optionally, the third crosslinking agent in the third crosslinking agent solution has at least one active group capable of reacting with amino group(s) and one hydrophilic group.

Optionally, the group capable of reacting with amino group(s) includes an ester bond or an epoxy group, and the hydrophilic group includes an alcoholic hydroxyl group or an ether bond.

Optionally, the third crosslinking agent is polyethylene glycol diglycidyl ether.

Catalysts can be added during the post-crosslinking to accelerate the reaction, and the catalysts can be selected from methylimidazole, triethylamine, and tetramethylethylenediamine.

Polyethylene glycol diglycidyl ether as the third crosslinking agent reacts with the residual amino groups to form a cross-linked network through polyethylene glycol. On the one hand, the high symmetry of the polyethylene glycol chain improves the high elasticity of the biological tissue, so that the ability of the biological material to deform elastically can be fully restored. On the other hand, water is a benign solvent for the polyethylene glycol, so that after being soaked in water, the biological material exhibits a high elasticity, further improving the crease resistance thereof.

Optionally, the solvent for preparing the third crosslinking agent solution is a conventional solvent for treating biological material, which will not destroy the structure of the biological material, such as water, PBS or physiological saline etc.. The third crosslinking agent in the third crosslinking agent solution has a mass percentage concentration ranging from 0.01% to 2%.

Optionally, the method includes exposing the biological material into the third crosslinking agent solution for 2 h to 48 h.

Optionally, the method includes exposing the biological material into the third crosslinking agent solution by static contact or dynamic contact.

Optionally, all the above methods with various treating sequences further include a reduction step of exposing the post-cross-linked biological material into a reductant solution. The amino groups react with the aldehyde groups to form schiff-base chemical bonds, but the schiff-base chemical bonds are reversible. By means of reduction, the schiff-base chemical bonds can be converted into stable carbon-nitrogen single bonds, avoiding the chemical bond exchange reaction during the crimping process.

Optionally, the reductant in the reductant solution is sodium cyanoborohydride or sodium borohydride. The solvent for preparing the reductant solution is a conventional solvent for treating biological material, which will not destroy the structure of the biological material, such as water, PBS or physiological saline etc.. The reductant has a mass percentage concentration ranging from 0.01% to 2%, and the method includes exposing the biological material into the reductant solution for 1 to 12 h by static contact or dynamic contact.

Optionally, the reduced biological material is subjected to the (d) dehydration or drying treatment.

Optionally, the dehydration uses ethanol dehydration; the drying treatment uses freeze drying.

Crease resistant dehydrated cross-linked biological materials prepared by the above methods are provided.

Optionally, the dehydrated cross-linked biological material has a maximum breaking force n ranging from 25 to 30 N.

Optionally, the maximum breaking force of the dehydrated cross-linked biological material is configured to be tested after hydration.

Optionally, the maximum breaking force is tested by: after the dehydrated cross-linked biological material is hydrated, it is cut into 1 cm * 5 cm pericardium strips, and measured by a universal tensile machine.

The hydration of the dehydrated cross-linked biological material refers to that the dehydrated cross-linked biological material reabsorbing water, for example, being soaked in a physiological saline solution.

Optionally, the dehydrated cross-linked biological material is configured to be crimped and maintained for at least 72 hours and become flattened without creases.

Optionally, the dehydrated cross-linked biological material is crimped and compressed in a 3 * 3 cm sheet into a 1 ml syringe, placed in an oven at 40 °C for 3 days, then released into physiological saline solution, and becomes flattened without creases.

The biological tissue prepared according to the present disclosure can be used for interventional biological valve, for example, through minimally invasive intervention, and can alternatively be used for surgical biological valve, for example, being implanted through surgery.

A biological valve includes a stent and a valve provided on the stent, and the valve is the crease resistant dehydrated cross-linked biological material disclosed here.

The biological valve can be an interventional valve through minimally invasive intervention, or an implantable valve through surgery.

An interventional system includes a heart valve and a delivery catheter, the heart valve is crimped and delivered by the delivery catheter, and the heart valve is the biological valve described disclosed here.

Compared with the prior art, the present invention has at least one of the following beneficial effects:
(1) The glycosyl groups in the biological material are opened in situ and form part of the cross-linked network. The elasticity and water absorption of the obtained cross-linked biological material are significantly improved, and the biological material, although being crimped for a long time in the delivery device, has no obvious creases after rehydration. Further, the mucopolysaccharide molecules are part of the biological material, which open in situ and form part of the cross-linked network, solving the problems of both crease recovery and immunogenicity.
(2) The residual active groups of the first crosslinking agent and the second crosslinking agent can react with each other, omitting the step of blocking the residual active groups.
(3) In the end-capping step, the capping substance is a polyamino substance. The polyamino substance includes at least two amino groups, some of which react with the residual aldehyde groups, with the remaining amino groups as residual amino groups. The polyamino substance has a high content of amino groups and thus a high content of residual amino groups, so that there are many sites for crosslinking with the third crosslinking agent in the post-crosslinking step, and the resulted cross-linked network can further enhance the elasticity of the biological material.
(4) The oxidant oxidizes the ortho-hydroxyl groups of mucopolysaccharides in the pericardium into aldehyde groups, thereby connecting the polysaccharides to the collagen fibers; and then by crosslinking with polyamines, a one-piece network is formed where cross-linking occurs between the polysaccharides, and between the polysaccharide and the collagen fiber.
(5) Polyethylene glycol diglycidyl ether as the third crosslinking agent reacts with the residual amino groups to form a cross-linked network through polyethylene glycol. The high symmetry of the polyethylene glycol chain improves the high elasticity of the biological tissue. Water is a benign solvent for the polyethylene glycol, so that after being soaked in water, the biological material exhibits a high elasticity, further improving the crease resistance thereof.

### BRIEF DESCRIPTION OF DRAWINGS

FIG. 1 is a schematic diagram of oxidation;
FIG. 2 is a schematic diagram of crosslinking with a first crosslinking agent and a second crosslinking agent ;
FIG. 3 is a schematic diagram of oxidation, crosslinking with the first crosslinking agent and crosslinking with the second crosslinking agent in sequence;
FIG. 4 is a flow chart of (a) oxidation, (b) first crosslinking, (c) second crosslinking and (d) dehydration performed in sequence;
FIG. 5 is a flow chart of (c) second crosslinking, (a) oxidation, (b) first crosslinking and (d) dehydration in sequence (not part of the invention);
FIG. 6 is a flow chart of (a) oxidation, b) first crosslinking, (c) second crosslinking and (d) dehydration performed in sequence;
FIG. 7 is a flowchart of Example 1;
FIG. 8 is a graph comparing the maximum breaking force of the cross-linked pericardium prepared in Example 1 and the conventional glutaraldehyde cross-linked pericardium;
FIG. 9 is a graph showing the result of the crease resistance test of the cross-linked pericardium prepared in Example 1;
FIG. 10 is a graph comparing the maximum breaking force of the cross-linked pericardium prepared in Example 2 and the conventional glutaraldehyde cross-linked pericardium;
FIG. 11 is a graph showing the result of the crease resistance test of the cross-linked pericardium prepared in Example 2;
FIG. 12 is a graph comparing the maximum breaking force of the cross-linked pericardium prepared in Example 4 and the conventional glutaraldehyde cross-linked pericardium; and
FIG. 13 is a graph showing the result of the crease resistance test of the cross-linked pericardium prepared in Example 4.

### DDESCRIPTION OF EMBODIMENTS

The technical solutions according to the embodiments of the present disclosure will be described clearly and fully in combination with the drawings according to the embodiments of the present disclosure. Obviously, the described embodiments are not all embodiments of the present disclosure, but only part of the embodiments of the present disclosure. Based on the disclosed embodiments, all other embodiments obtained by those skilled in the art without creative work fall into the scope of this invention.

Unless otherwise defined, all technical and scientific terms used herein have the same meaning as commonly understood by a person skilled in the art. The terms in the description of the present disclosure are used to describe specific embodiments, and not to limit the present disclosure.

In the first embodiment, a biological material is subjected to oxidation, crosslinking and dehydration in sequence. The crosslinking includes a first crosslinking and a second crosslinking performed in sequence. During the oxidation, an oxidant oxidizes ortho-hydroxyl groups of mucopolysaccharides of the biological material into aldehyde groups, thereby connecting the polysaccharides to the collagen fibers; then by means of crosslinking, the polysaccharides and the collagen fibers are respectively cross-linked to form a one-piece cross-linked network, and within the final network, cross-linking occurs between polysaccharides, polysaccharide and collagen fiber, and collagen fibers.

After the second crosslinking, end-capping, post-crosslinking and reduction can be performed, followed by dehydration. In the end-capping, the residual active groups of the second crosslinking agent are blocked, in the post-crosslinking, the residual active groups of the capping substance are cross-linked, and in the reduction, the schiff-base chemical bonds are converted into stable carbon-nitrogen single bonds.

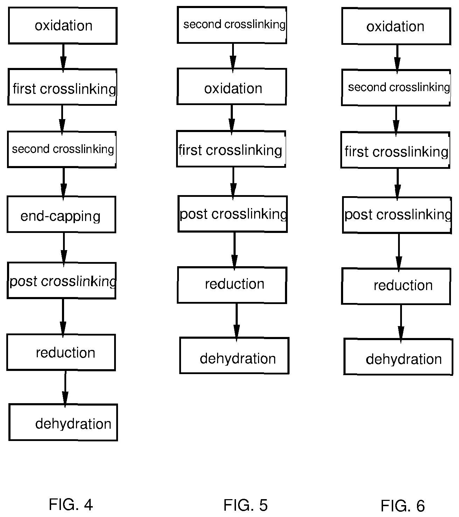

The flow chart of this embodiment is shown in FIG. 4, where the biological material is sequentially subjected to oxidation, first crosslinking, second crosslinking, end-capping, post-crosslinking, reduction and dehydration.

### Oxidation:

Take a fresh pericardium, and cut and hang the same on a board, wherein the pericardium is a pig pericardium or a bovine pericardium. Then the pericardium is exposed to an oxidant solution by soaking or spraying, wherein the oxidant can be sodium periodate, and the mass percentage concentration thereof in the oxidant solution is 0.1% to 1%, and the oxidation time is 1 to 12 hours.

After oxidation, the ortho-hydroxyl groups of the mucopolysaccharides of the pericardium are oxidized into aldehyde groups, and the aldehyde groups react with the amino groups of the collagen fibers, thereby connecting the mucopolysaccharides to the collagen fibers.

### First crosslinking:

After the mucopolysaccharides are connected to the collagen fibers, the mucopolysaccharides are cross-linked through aldehyde groups. The first crosslinking agent should have a group that can react with the aldehyde group, and preferably uses a bisamino compound. The mucopolysaccharides are cross-linked with each other through the reaction of the amino groups with the aldehyde groups.

The bisamino compound can be selected from lysine, ethylenediamine or hexamethylenediamine. The mass percentage concentration of the bisamino compound in the bisamino compound solution is 0.05% to 5%, the exposure time of the biological material in the bisamino compound solution is 0.5 to 12h, and the exposure method can be soaking or spraying.

### Second crosslinking:

The collagen fibers of the biological material are cross-linked by a second crosslinking agent, and the second crosslinking agent can be glutaraldehyde. In this step, the biological material is exposed to the glutaraldehyde solution by soaking or spraying. The mass percentage concentration of glutaraldehyde in the glutaraldehyde solution is 0.05% to 25%, the crosslinking time is 6 h to 3 weeks, and the crosslinking temperature is 0 to 37 °C.

### End-capping:

After crosslinking using the glutaraldehyde, there are residual aldehyde groups in the biological material. In this step, end-capping is performed through a capping substance. The capping substance is preferably a polyamino substance, and the polyamino substance can be selected from polyethyleneimine, polylysine, ε-polylysine, lysine, hexamethylenediamine, etc.

In this step, the biological material is exposed to the polyamino substance solution. The mass percentage concentration of the polyamino substance in the polyamino substance solution is 0.5% to 10%, the exposure time is 0.5 h to 12 h, and the exposure method can be soaking or spraying.

### Third crosslinking:

In the previous end-capping step, the polyamino substance is used as the capping substance. The residual amino groups are then cross-linked by a third crosslinking agent in this step. The third crosslinking agent is polyethylene glycol diglycidyl ether. The cross-linked network formed by polyethylene glycol improves the high elasticity of the pericardium. On the one hand, the high symmetry of the polyethylene glycol chain improves the high elasticity of the biological tissue, so that the ability of the biological material to deform elastically can be fully restored. On the other hand, water is a benign solvent for the polyethylene glycol, so that after being soaked in water, the biological material exhibits a high elasticity, further improving the crease resistance thereof.

The mass percentage concentration of the third crosslinking agent in the third crosslinking agent solution is 0.01% to 2%, and the crosslinking time is 10 to 48 h.

### Reduction:

The amino groups react with the aldehyde groups to form schiff-base chemical bonds, but the schiff-base chemical bonds are reversible. By means of reduction, the schiff-base chemical bonds can be converted into stable carbon-nitrogen single bonds, avoiding the chemical bond exchange reaction during the crimping process.

In this step, the biological material is exposed to a reductant solution, and the reductant can be sodium cyanoborohydride or sodium borohydride. The mass percentage concentration of the reductant in the reductant solution is 0.01% to 2%, the exposure time is 1 to 12 hours, and the exposure method can be soaking or spraying.

### Dehydration:

After soaking in glycerol, ethanol dehydration or freeze drying is performed.

In the second embodiment (not part of the invention), a biological material is subjected to a second crosslinking, oxidation, first crosslinking and dehydration in sequence. During the second crosslinking, the collagen fibers of the biological material are cross-linked with each other. During the oxidation, an oxidant oxidizes ortho-hydroxyl groups of mucopolysaccharides of the biological material into aldehyde groups, thereby connecting the polysaccharides to the collagen fibers. Then, during the first crosslinking, polysaccharides and polysaccharides are cross-linked with each other. In the final network, cross-linking occurs between the collagen fiber and the collagen fiber, the polysaccharide and the collagen fiber, and the polysaccharide and the polysaccharide.

After the first crosslinking, post-crosslinking and reduction can be performed, followed by dehydration, without end-capping. In the post-crosslinking, the residual active groups of the first crosslinking agent are cross-linked, and in the reduction, the schiff-base chemical bonds are converted into stable carbon-nitrogen single bonds.

The flow chart of this embodiment is shown in FIG. 5, where the biological material is sequentially subjected to second crosslinking, oxidation, first crosslinking, post-crosslinking, reduction and dehydration.

### Second crosslinking:

The collagen fibers of the biological material are cross-linked by a second crosslinking agent, and the second crosslinking agent can be glutaraldehyde. In this step, the biological material is exposed to the glutaraldehyde solution by soaking or spraying. The mass percentage concentration of glutaraldehyde in the glutaraldehyde solution is 0.05% to 25%, the crosslinking time is 6 h to 3 weeks, and the crosslinking temperature is 0 to 37 °C.

### Oxidation:

Take a fresh pericardium, and cut and hang the same on a board, wherein the pericardium is a pig pericardium or a bovine pericardium. Then the pericardium is exposed to an oxidant solution by soaking or spraying, wherein the oxidant can be sodium periodate, and the mass percentage concentration thereof in the oxidant solution is 0.1% to 1%, and the oxidation time is 1 to 12 hours.

After oxidation, the ortho-hydroxyl groups of the mucopolysaccharides of the pericardium are oxidized into aldehyde groups, and the aldehyde groups react with the amino groups of the collagen fibers, thereby connecting the mucopolysaccharides to the collagen fibers.

### First crosslinking:

After the mucopolysaccharides are connected to the collagen fibers, the mucopolysaccharides are cross-linked through aldehyde groups. The first crosslinking agent should have a group that can react with the aldehyde group, and preferably uses a polyamino substance, for example, being selected from lysine, ethylenediamine or hexamethylenediamine, polyethyleneimine, polylysine, or ε-polylysine. The mucopolysaccharides are cross-linked with each other through the reaction of the amino groups with the aldehyde groups.

The mass percentage concentration of the polyamino substances in the polyamino substance solution is 0.5% to 10%, the exposure time of the biological material in the polyamino substance solution is 0.5 to 12 h, and the exposure method can be soaking or spraying.

### Post-crosslinking:

In the previous first crosslinking step, the polyamino substance is used as the first crosslinking agent. The residual amino groups are then cross-linked by a third crosslinking agent in this step. The third crosslinking agent is polyethylene glycol diglycidyl ether. The cross-linked network formed by polyethylene glycol improves the high elasticity of the pericardium. On the one hand, the high symmetry of the polyethylene glycol chain improves the high elasticity of the biological tissue, so that the ability of the biological material to deform elastically can be fully restored. On the other hand, water is a benign solvent for the polyethylene glycol, so that after being soaked in water, the biological material exhibits a high elasticity, further improving the crease resistance thereof.

The mass percentage concentration of the third crosslinking agent in the third crosslinking agent solution is 0.01% to 2%, and the crosslinking time is 10 to 48 h.

### Reduction:

The amino groups react with the aldehyde groups to form schiff-base chemical bonds, but the schiff-base chemical bonds are reversible. By means of reduction, the schiff-base chemical bonds can be converted into stable carbon-nitrogen single bonds, avoiding the chemical bond exchange reaction during the crimping process.

In this step, the biological material is exposed to a reductant solution, and the reductant can be sodium cyanoborohydride or sodium borohydride. The mass percentage concentration of the reductant in the reductant solution is 0.01% to 2%, the exposure time is 1 to 12 hours, and the exposure method can be soaking or spraying.

### Dehydration:

After soaking in glycerol, ethanol dehydration or freeze drying is performed.

In the third embodiment, a biological material is subjected to oxidation, second crosslinking, first crosslinking and dehydration in sequence. In the present invention said sequence is carried out by exposing the biological material into a solution containing the oxidant, a second crosslinking agent solution and a first crosslinking agent solution. During the oxidation, ar oxidant oxidizes ortho-hydroxyl groups of mucopolysaccharides of the biological material into aldehyde groups, thereby connecting the polysaccharides to the collagen fibers. During the second crosslinking, the collagen fibers of the biological material are cross-linked with each other. Then, during the first crosslinking, polysaccharides and polysaccharides are cross-linked with each other. In the final network, cross-linking occurs between the collagen fiber and the collagen fiber, the polysaccharide and the collagen fiber, and the polysaccharide and the polysaccharide.

After the first crosslinking, post-crosslinking and reduction can be performed, followed by dehydration, without end-capping. In the post-crosslinking, the residual active groups of the first crosslinking agent are cross-linked, and in the reduction, the schiff-base chemical bonds are converted into stable carbon-nitrogen single bonds.

The flow chart of this embodiment is shown in FIG. 6, where the biological material is sequentially subjected to oxidation, second crosslinking, first crosslinking, post-crosslinking, reduction and dehydration.

### Oxidation:

Take a fresh pericardium, and cut and hang the same on a board, wherein the pericardium is a pig pericardium or a bovine pericardium. Then the pericardium is exposed to an oxidant solution by soaking or spraying, wherein the oxidant can be sodium periodate, and the mass percentage concentration thereof in the oxidant solution is 0.1% to 1%, and the oxidation time is 1 to 12 hours.

After oxidation, the ortho-hydroxyl groups of the mucopolysaccharides of the pericardium are oxidized into aldehyde groups, and the aldehyde groups react with the amino groups of the collagen fibers, thereby connecting the mucopolysaccharides to the collagen fibers.

### Second crosslinking:

The collagen fibers of the biological material are cross-linked by a second crosslinking agent, and the second crosslinking agent can be glutaraldehyde. In this step, the biological material is exposed to the glutaraldehyde solution by soaking or spraying. The mass percentage concentration of glutaraldehyde in the glutaraldehyde solution is 0.05% to 25%, the crosslinking time is 6 h to 3 weeks, and the crosslinking temperature is 0 to 37 °C.

### First crosslinking:

After the mucopolysaccharides are connected to the collagen fibers, the mucopolysaccharides are cross-linked through aldehyde groups. The first crosslinking agent should have a group that can react with the aldehyde group, and preferably uses a polyamino substance, for example, being selected from lysine, ethylenediamine or hexamethylenediamine, polyethyleneimine, polylysine, or ε-polylysine. The mucopolysaccharides are cross-linked with each other through the reaction of the amino groups with the aldehyde groups.

The mass percentage concentration of the polyamino substances in the polyamino substance solution is 0.5% to 10%, the exposure time of the biological material in the polyamino substance solution is 0.5 to 12 h, and the exposure method can be soaking or spraying.

### Post-crosslinking:

In the previous first crosslinking step, the polyamino substance is used as the first crosslinking agent. The residual amino groups are then cross-linked by a third crosslinking agent in this step. The third crosslinking agent is polyethylene glycol diglycidyl ether. The cross-linked network formed by polyethylene glycol improves the high elasticity of the pericardium. On the one hand, the high symmetry of the polyethylene glycol chain improves the high elasticity of the biological tissue, so that the ability of the biological material to deform elastically can be fully restored. On the other hand, water is a benign solvent for the polyethylene glycol, so that after being soaked in water, the biological material exhibits a high elasticity, further improving the crease resistance thereof.

The mass percentage concentration of the third crosslinking agent in the third crosslinking agent solution is 0.01% to 2%, and the crosslinking time is 10 to 48 h.

### Reduction:

The amino groups react with the aldehyde groups to form schiff-base chemical bonds, but the schiff-base chemical bonds are reversible. By means of reduction, the schiff-base chemical bonds can be converted into stable carbon-nitrogen single bonds, avoiding the chemical bond exchange reaction during the crimping process.

In this step, the biological material is exposed to a reductant solution, and the reductant can be sodium cyanoborohydride or sodium borohydride. The mass percentage concentration of the reductant in the reductant solution is 0.01% to 2%, the exposure time is 1 to 12 hours, and the exposure method can be soaking or spraying.

### Dehydration:

After soaking in glycerol, ethanol dehydration or freeze drying is performed.

In the above three embodiments, the solvent for preparing the solution uses a conventional solvent for treating a biological material, which will not negatively affect the biological material, for example, water, PBS or physiological saline.

Specific examples will be described below:
In the following examples, the concentrations expressed as percentage represent mass percentage concentrations unless otherwise specified; the solutions mentioned in the following examples are all prepared by PBS unless otherwise specified.

### Example 1

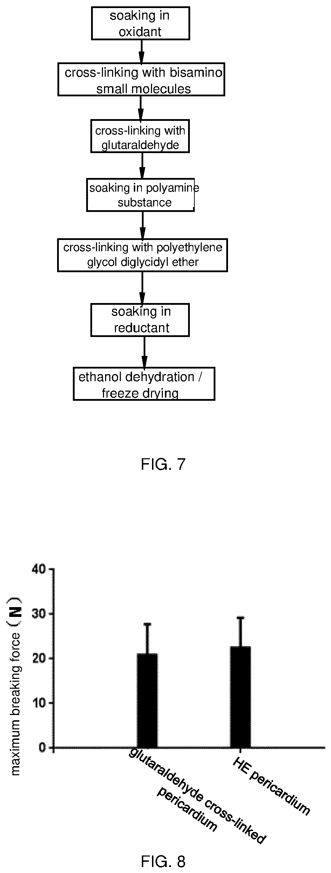

Referring to the flow chart shown in FIG. 7, a porcine pericardium was soaked in 1% sodium periodate solution at room temperature for 2 h, soaked in 2% lysine aqueous solution for 2 h, subsequently, cross-linked in 0.05% glutaraldehyde solution for 5 days, washed with 0.9% physiological saline, soaked in 2% ε-polylysine aqueous solution for 2 h, soaked in 2% polyethylene glycol diglycidyl ether aqueous solution added with 0.1% tetramethylethylenediamine catalyst for 24 h, washed with 0.9% physiological saline three times, soaked in 1% sodium cyanoborohydride aqueous solution for 2 h, soaked in 40% glycerol aqueous solution after being washed 3 times, and then freeze-dried.

The pericardium prepared in Example 1 was subjected to breaking force test and water absorption and flattening test.

Breaking force test: after the dry pericardium was hydrated, it was cut into 1 * 5 cm strips, and the maximum breaking force was measured by a universal tensile machine. A conventional glutaraldehyde cross-linked pericardium was used as a control group. The results are shown in FIG. 8. The breaking force of the pericardium prepared in Example 1 is 25 to 30 N, which is significantly greater than the conventional glutaraldehyde cross-linked pericardium.

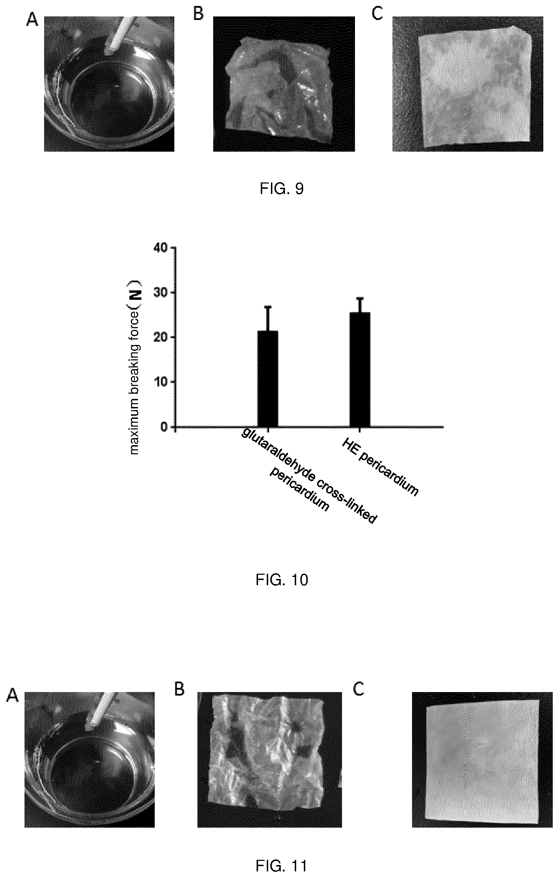

Water absorption and flattening test: 3 * 3 cm pericardium sheets were crimped and compressed into a 1 ml syringe (as shown in FIG. 9A), placed in an oven at 40 °C for 3 days, then released into physiological saline solution. Observe the flattening of the sheets. The results are shown in FIG. 9B and FIG. 9C, where FIG. 9B shows a conventional glutaraldehyde cross-linked dry pericardium which has a lot of creases, and FIG. 9C shows the HE dry pericardium (prepared in Example 1) which has no obvious visible crease after being released and soaked in water.

### Example 2 (not part of the invention)

Referring to the flow chart shown in FIG. 5, a porcine pericardium was cross-linked in 0.05% glutaraldehyde solution for 5 days, warshed with 0.9% physiological saline, soaked in 1% sodium periodate solution at room temperature for 2 h, subsequently, soaked in 2% ε-polylysine solution for 2 h, soaked in 2% polyethylene glycol diglycidyl ether added with 0.1% catalyst for 24 h, washed with 0.9% physiological saline three times, soaked in 1% sodium cyanoborohydride solution for 2 h, soaked in 40% isopropanol solution after being washed 3 times, and then blow-dried or freeze-dried.

The breaking force test was the same as in Example 1. The results are shown in FIG. 10, which shows that the breaking force of the pericardium prepared in Example 2 was significantly greater than that of the conventional glutaraldehyde cross-linked pericardium.

Water absorption and flattening test: 3 * 3 cm pericardium sheets were crimped and compressed into a 1 ml syringe (as shown in FIG. 11A), placed in an oven at 40 °C for 3 days, then released into physiological saline solution. Observe the flattening of the sheets. The results are shown in FIG. 10B and FIG. 10C, where FIG. 10B shows a conventional glutaraldehyde cross-linked dry pericardium which has a lot of creases, and FIG. 10C shows the HE dry pericardium (prepared in Example 2) which has no obvious visible crease after being released and soaked in water.

### Example 3

A porcine pericardium was soaked in 1% sodium periodate solution for 2 h at room temperature, soaked in 2% lysine solution for 2 h, subsequently, cross-linked in 0.05% glutaraldehyde solution for 5 days, washed with 0.9% physiological saline, soaked in 2% bisamino polyethylene glycol solution for 24 h, washed with 0.9% physiological saline three times, soaked in 1% sodium cyanoborohydride solution for 2 h, soaked in 40% glycerol aqueous solution after being washed 3 times, and then freeze-dried.

### Example 4 (not part of the invention)

A porcine pericardium was soaked in 1% sodium periodate solution at room temperature for 2 h, cross-linked in 0.05% glutaraldehyde solution for 5 days and washed with 0.9% physiological saline, soaked in 2% polyethyleneimine solution for 2 h, subsequently, soaked in 2% bisamino polyethylene glycol aqueous solution for 24 h, washed with 0.9% physiological saline three times, soaked in 1% sodium cyanoborohydride solution for 2 h, soaked in 60% glycerin aqueous solution after being washed 3 times, and then blow-dried.

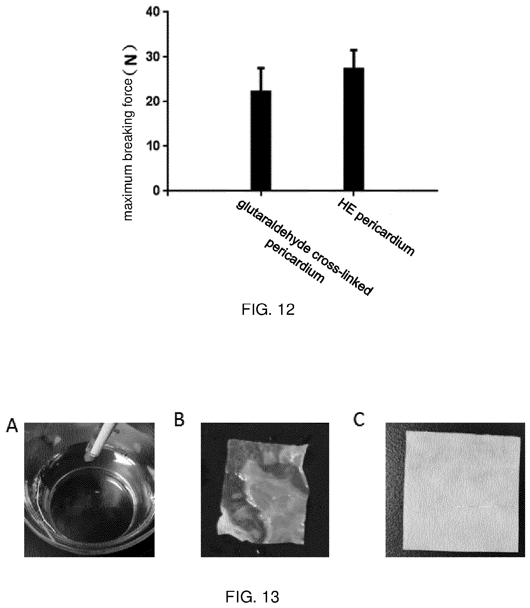

The breaking force test was the same as in Example 1. The results are shown in FIG. 12, which shows that the breaking force of the pericardium prepared in Example 4 was significantly greater than that of the conventional glutaraldehyde cross-linked pericardium.

Water absorption and flattening test: 3 * 3 cm pericardium sheets were crimped and compressed into a 1 ml syringe (as shown in FIG. 13A), placed in an oven at 40 °C for 3 days, then released into physiological saline solution. Observe the flattening of the sheets. The results are shown in FIG. 13B and FIG. 13C, where FIG. 13B shows a conventional glutaraldehyde cross-linked dry pericardium which has a lot of creases, and FIG. 13C shows the HE dry pericardium (prepared in Example 4) which has no obvious visible crease after being released and soaked in water.

The above-described embodiments only illustrate several embodiments of the present disclosure, and the description thereof is specific and detail, but should not be construed as limiting the scope of the patent disclosure.

## Claims

1. A crease resistant dehydrated biological material prepared by composite crosslinking, using a preparation method comprising:
performing (a) oxidation, (b) first crosslinking, (c) second crosslinking and (d) dehydration on a biological material, wherein (b) is performed after (a), and (a), (b) and (c) are all performed prior to (d); and
wherein the oxidation uses an oxidant capable of converting hydroxyl groups of mucopolysaccharides of the biological material into aldehyde groups, the first crosslinking uses a first crosslinking agent capable of causing cross-linking between the aldehyde groups of the mucopolysaccharides to form a cross-linked network, and the second crosslinking uses a second crosslinking agent capable of causing cross-linking between collagen fibers of the biological material;
wherein the method comprising performing (a) oxidation, (b) first crosslinking and (c) second crosslinking on the biological material in sequence: exposing the biological material after oxidation to a first crosslinking agent solution and a second crosslinking agent solution, or
wherein the method comprising performing (a) oxidation, (c) second crosslinking and (b) first crosslinking on the biological material in sequence: exposing the biological material into a solution containing the oxidant, a second crosslinking agent solution and a first crosslinking agent solution.

2. The crease resistant dehydrated biological material according to claim 1, wherein the biological material is originated from pig, cattle, horse or sheep, and selected from pericardium, heart valve, blood vessel, ligament, muscle, intestine or skin.

3. The crease resistant dehydrated biological material according to claim 1, wherein the oxidation comprises exposing the biological material into a solution containing the oxidant.

4. The crease resistant dehydrated biological material according to claim 3, wherein the hydroxyl group is an ortho-hydroxyl group from the mucopolysaccharide.

5. The crease resistant dehydrated biological material according to claim 3, wherein the oxidant is sodium periodate.

6. The crease resistant dehydrated biological material according to claim 3, wherein the oxidant in the solution containing the oxidant has a mass percentage concentration ranging from 0.1% to 1%, and the method comprising exposing the biological material into the solution containing the oxidant for 1 to 12 hours by static contact or dynamic contact.

7. The crease resistant dehydrated biological material according to claim 1, wherein when the method comprises performing (a) oxidation, (b) first crosslinking and (c) second crosslinking on the biological material in sequence, the first crosslinking agent has at least two active groups capable of reacting with the aldehyde groups, and the second crosslinking agent is different from the first crosslinking agent and capable of reacting with the active groups of the first crosslinking agent.

8. The crease resistant dehydrated biological material according to claim 7, wherein the first crosslinking agent is a small molecular substance having at least two amino groups.

9. The crease resistant dehydrated biological material according to claim 8, wherein the first crosslinking agent is lysine, ethylenediamine or hexamethylenediamine.

10. The crease resistant dehydrated biological material according to claim 1, wherein the method further comprising end-capping of exposing the cross-linked biological material into a solution containing a capping substance to block residual active groups from the second crosslinking agent.

11. The crease resistant dehydrated biological material according to claim 1, wherein when the method comprises performing (a) oxidation, (c) second crosslinking and (b) first crosslinking on the biological material in sequence, the method further comprising post-crosslinking of exposing the treated with the first crosslinking agent into a third crosslinking agent solution to crosslink residual active groups from the first crosslinking agent.

12. A biological valve, comprising a stent and a valve provided on the stent, wherein the valve is the crease resistant dehydrated biological material according to any one of claims 1 to 11.

## Patentansprüche

1. Ein knitterbeständiges dehydriertes biologisches Material, hergestellt durch kombinierte Vernetzung unter Verwendung eines Herstellungsverfahrens, das Folgendes umfasst:
das Durchführen von (a) Oxidation, (b) erster Vernetzung, (c) zweiter Vernetzung und (d) Dehydrierung an einem biologischen Material, wobei (b) nach (a) durchgeführt wird und (a), (b) und (c) sämtlich vor (d) durchgeführt werden; und
wobei bei der Oxidation ein Oxidationsmittel verwendet wird, das Hydroxylgruppen von Mucopolysacchariden des biologischen Materials in Aldehydgruppen umwandeln kann, bei der ersten Vernetzung ein erstes Vernetzungsmittel verwendet wird, das eine Vernetzung zwischen den Aldehydgruppen der Mucopolysaccharide unter Bildung eines vernetzten Netzwerks bewirken kann, und bei der zweiten Vernetzung ein zweites Vernetzungsmittel verwendet wird, das eine Vernetzung zwischen Kollagenfasern des biologischen Materials bewirken kann;
wobei das Verfahren das Durchführen von (a) Oxidation, (b) erster Vernetzung und (c) zweiter Vernetzung an dem biologischen Material in dieser Reihenfolge umfasst: das Aussetzen des biologischen Materials nach der Oxidation gegenüber einer Lösung eines ersten Vernetzungsmittels und einer Lösung eines zweiten Vernetzungsmittels, oder
wobei das Verfahren das Durchführen von (a) Oxidation, (c) zweiter Vernetzung und (b) erster Vernetzung an dem biologischen Material in dieser Reihenfolge umfasst: das Aussetzen des biologischen Materials gegenüber einer das Oxidationsmittel enthaltenden Lösung, einer Lösung eines zweiten Vernetzungsmittels und einer Lösung eines ersten Vernetzungsmittels.

2. Das knitterbeständige dehydrierte biologische Material nach Anspruch 1, wobei das biologische Material von Schwein, Rind, Pferd oder Schaf stammt und aus Perikard, Herzklappe, Blutgefäß, Band, Muskel, Darm oder Haut ausgewählt ist.

3. Das knitterbeständige dehydrierte biologische Material nach Anspruch 1, wobei die Oxidation das Aussetzen des biologischen Materials gegenüber einer das Oxidationsmittel enthaltenden Lösung umfasst.

4. Das knitterbeständige dehydrierte biologische Material nach Anspruch 3, wobei die Hydroxylgruppe eine ortho-Hydroxylgruppe aus dem Mucopolysaccharid ist.

5. Das knitterbeständige dehydrierte biologische Material nach Anspruch 3, wobei das Oxidationsmittel Natriumperiodat ist.

6. Das knitterbeständige dehydrierte biologische Material nach Anspruch 3, wobei das Oxidationsmittel in der das Oxidationsmittel enthaltenden Lösung eine Massenprozentkonzentration im Bereich von 0,1 % bis 1 % aufweist und das Verfahren das Aussetzen des biologischen Materials gegenüber der das Oxidationsmittel enthaltenden Lösung für 1 bis 12 Stunden durch statischen Kontakt oder dynamischen Kontakt umfasst.

7. Das knitterbeständige dehydrierte biologische Material nach Anspruch 1, wobei, wenn das Verfahren das Durchführen von (a) Oxidation, (b) erster Vernetzung und (c) zweiter Vernetzung an dem biologischen Material in dieser Reihenfolge umfasst, das erste Vernetzungsmittel mindestens zwei aktive Gruppen aufweist, die mit den Aldehydgruppen reagieren können, und das zweite Vernetzungsmittel von dem ersten Vernetzungsmittel verschieden ist und mit den aktiven Gruppen des ersten Vernetzungsmittels reagieren kann.

8. Das knitterbeständige dehydrierte biologische Material nach Anspruch 7, wobei das erste Vernetzungsmittel eine niedermolekulare Substanz mit mindestens zwei Aminogruppen ist.

9. Das knitterbeständige dehydrierte biologische Material nach Anspruch 8, wobei das erste Vernetzungsmittel Lysin, Ethylendiamin oder Hexamethylendiamin ist.

10. Das knitterbeständige dehydrierte biologische Material nach Anspruch 1, wobei das Verfahren ferner einen Endgruppenverschluss durch Aussetzen des vernetzten biologischen Materials gegenüber einer einen Endgruppenverschlussstoff enthaltenden Lösung umfasst, um verbleibende aktive Gruppen des zweiten Vernetzungsmittels zu blockieren.

11. Das knitterbeständige dehydrierte biologische Material nach Anspruch 1, wobei, wenn das Verfahren das Durchführen von (a) Oxidation, (c) zweiter Vernetzung und (b) erster Vernetzung an dem biologischen Material in dieser Reihenfolge umfasst, das Verfahren ferner eine Nachvernetzung durch Aussetzen des mit dem ersten Vernetzungsmittel behandelten biologischen Materials gegenüber einer Lösung eines dritten Vernetzungsmittels umfasst, um verbleibende aktive Gruppen des ersten Vernetzungsmittels zu vernetzen.

12. Eine biologische Klappe, die einen Stent und eine an dem Stent vorgesehene Klappe umfasst, wobei die Klappe das knitterbeständige dehydrierte biologische Material nach einem der Ansprüche 1 bis 11 ist.

## Revendications

1. Matériau biologique déshydraté résistant au froissement préparé par réticulation composite, en utilisant un procédé de préparation comprenant :
le fait de réaliser (a) une oxydation, (b) une première réticulation, (c) une seconde réticulation et (d) une déshydratation sur un matériau biologique, où l'étape (b) est réalisée après l'étape (a), et les étapes (a), (b) et (c) sont toutes réalisées avant l'étape (d) ; et
où l'oxydation utilise un oxydant capable de convertir des groupements hydroxyle de mucopolysaccharides du matériau biologique en groupements aldéhyde, la première réticulation utilise un premier agent de réticulation capable de provoquer une réticulation entre les groupements aldéhyde des mucopolysaccharides pour former un réseau réticulé, et la seconde réticulation utilise un second agent de réticulation capable de provoquer une réticulation entre les fibres de collagène du matériau biologique ;
où le procédé comprend le fait de réaliser (a) une oxydation, (b) une première réticulation et (c) une seconde réticulation sur le matériau biologique successivement : en exposant le matériau biologique après oxydation à une première solution d'agent de réticulation et à une seconde solution d'agent de réticulation, ou
où le procédé comprend le fait de réaliser (a) une oxydation, (c) une seconde réticulation et (b) une première réticulation sur le matériau biologique successivement : en exposant le matériau biologique dans une solution contenant l'oxydant, une seconde solution d'agent de réticulation et une première solution d'agent de réticulation.

2. Matériau biologique déshydraté résistant au froissement selon la revendication 1, où le matériau biologique est issu de porc, de bovin, de cheval ou de mouton, et sélectionné parmi un péricarde, un valvule cardiaque, un vaisseau sanguin, un ligament, un muscle, un intestin ou de la peau.

3. Matériau biologique déshydraté résistant au froissement selon la revendication 1, où l'oxydation comprend le fait d'exposer le matériau biologique dans une solution contenant l'oxydant.

4. Matériau biologique déshydraté résistant au froissement selon la revendication 3, où le groupement hydroxyle est un groupement ortho-hydroxyle du mucopolysaccharide.

5. Matériau biologique déshydraté résistant au froissement selon la revendication 3, où l'oxydant est le periodate de sodium.

6. Matériau biologique déshydraté résistant au froissement selon la revendication 3, où l'oxydant dans la solution contenant l'oxydant présente une concentration massique en pourcentage allant de 0,1 % à 1 %, et le procédé comprenant le fait d'exposer le matériau biologique dans la solution contenant l'oxydant pendant 1 à 12 heures par contact statique ou contact dynamique.

7. Matériau biologique déshydraté résistant au froissement selon la revendication 1, où lorsque le procédé comprend le fait de réaliser (a) une oxydation, (b) une première réticulation et (c) une seconde réticulation sur le matériau biologique successivement, le premier agent de réticulation présente au moins deux groupements fonctionnels capables de réagir avec les groupements aldéhyde, et le second agent de réticulation est différent du premier agent de réticulation et est capable de réagir avec les groupements fonctionnels du premier agent de réticulation.

8. Matériau biologique déshydraté résistant au froissement selon la revendication 7, où le premier agent de réticulation est une petite substance moléculaire présentant au moins deux groupements amino.

9. Matériau biologique déshydraté résistant au froissement selon la revendication 8, où le premier agent de réticulation est la lysine, l'éthylènediamine ou l'hexaméthylènediamine.

10. Matériau biologique déshydraté résistant au froissement selon la revendication 1, où le procédé comprend en outre un blocage de groupements terminaux consistant à exposer le matériau biologique réticulé dans une solution contenant une substance de blocage pour bloquer des groupements fonctionnels résiduels du second agent de réticulation.

11. Matériau biologique déshydraté résistant au froissement selon la revendication 1, où lorsque le procédé comprend le fait de réaliser (a) une oxydation, (c) une seconde réticulation et (b) une première réticulation sur le matériau biologique successivement, le procédé comprend en outre une post-réticulation consistant à exposer le matériau traité avec le premier agent de réticulation dans une troisième solution d'agent de réticulation pour réticuler les groupements fonctionnels résiduels du premier agent de réticulation.

12. Valvule biologique, comprenant un stent et une valvule prévue sur le stent, où la valvule est le matériau biologique déshydraté résistant au froissement selon l'une quelconque des revendications 1 à 11.
